(19) Europäisches Patentamt / European Patent Office / Office européen des brevets

(11) EP 3 333 550 A1

(12) EUROPEAN PATENT APPLICATION

(43) Date of publication:
13.06.2018 Bulletin 2018/24

(51) Int Cl.:
G01F 1/34 (2006.01)     G01F 7/00 (2006.01)
G01F 1/46 (2006.01)

(21) Application number: 17184563.9

(22) Date of filing: 02.08.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 09.12.2016 JP 2016239071

(71) Applicant: Horiba, Ltd.
Kyoto-shi, Kyoto 601-8510 (JP)

(72) Inventor: FUKAMI, Shun
Kyoto, 601-8510 (JP)

(74) Representative: Müller Hoffmann & Partner
Patentanwälte mbB
St.-Martin-Strasse 58
81541 München (DE)

(54) DIFFERENTIAL PRESSURE FLOW METER, EXHAUST GAS ANALYSIS DEVICE AND FLOW RATE MEASUREMENT METHOD

(57) In order to make highly accurate flow rate measurements over a broad range, the present invention provides a differential pressure flow meter 2 that detects differential pressures in a fluid body flowing along a flow path, and calculates a flow rate of the fluid body from those differential pressures, and that includes at least two differential pressure detecting portions 22 and 23 that have mutually different measurement ranges.

Fig.2

EP 3 333 550 A1

**Description**

[Technical Field]

The present invention relates to a differential pressure flow meter, an exhaust gas analysis device that uses this differential pressure flow meter, and a flow rate measurement method that uses this differential pressure flow meter.

[Technical Background]

Among the tests performed on vehicles is an on-road running test in which exhaust gas emitted from the tail pipe of a vehicle is measured while this vehicle is running on a road.

In this on-road running test, as is shown in Patent document 1, various types of exhaust gas analyzers are installed in a vehicle. These exhaust gas analyzers sample the exhaust gas emitted from the vehicle's tail pipe and analyze each component contained in the exhaust gas. Moreover, for example, a Pitot tube flow meter that is used to measure the flow rate of exhaust gas is mounted in the tail pipe, and the emission mass of each component is calculated from the exhaust gas flow rate obtained by the flow meter and from the concentrations of each component obtained by the exhaust gas analyzers.

Here, the Pitot tube flow meter is provided integrally with an attachment pipe that is fitted onto the tail pipe. The larger the pipe diameter of the attachment pipe, the greater the flow rate measurement range of the Pitot tube flow meter. Furthermore, this Pitot tube flow meter is mounted so as to correspond to the tail pipe diameter and the actual exhaust gas flow rate.

However, because only one Pitot tube flow meter is provided in an attachment pipe, it is not possible to alter the Pitot tube flow meter, for example, during an on-road running test.

Moreover, in the case of a vehicle in which there is a large difference between the exhaust gas flow rate when there is a low level of exhaust such as, for example, when the vehicle is idling, and the exhaust gas flow rate when there is a high level of exhaust such as, for example, when the vehicle is accelerating rapidly or is traveling at high speed, it is not possible to obtain both of these flow rates from a single Pitot tube flow meter, and there are cases when the flow rate range does not cover at least one of the exhaust gas flow rate when there is a low level of exhaust and the exhaust gas flow rate when there is a high level of exhaust.

Furthermore, some modem vehicles are equipped with a tail pipe having a larger diameter than is required by the actual exhaust gas flow rate in order to improve the appearance of the vehicle. In cases such as this, if an attachment pipe that matches the tail pipe diameter is attached, then the Pitot tube flow meter that is used ends up having an excessively broad measurement range relative to the exhaust gas flow rate.

For these reasons, the accuracy of the exhaust gas flow rate measurement is adversely affected and, as a result, there are also considerable measurement errors in the results of the emission mass measurement of each component contained in the exhaust gas.

[Documents of the prior art]

[Patent documents]

[Patent document 1] Japanese Unexamined Patent Application (JP-A) No. 2004-144574

[Disclosure of the Invention]

[Problems to be Solved by the Invention]

The present invention was therefore conceived in order to solve the above-described problems, and it is a principal object thereof to make it possible to measure a broad range of flow rates with a high degree of accuracy.

[Means for Solving the Problem]

Namely, the differential pressure flow meter according to the present invention is a differential pressure flow meter that detects differential pressures in a fluid body flowing along a flow path, and calculates a flow rate of the fluid body from those differential pressures, and has at least two differential pressure detecting portions having mutually different measurement ranges. Note that the term 'mutually different measurement ranges' refers not only to two measurement ranges that have no mutually overlapping portions, but also to two measurement ranges that have partially overlapping portions.

If this type of structure is employed, then because at least two differential pressure detecting portions having mutually different measurement ranges are provided, it is possible to measure a broad range of flow rates with a high degree of accuracy.

It is desirable that the at least two differential pressure detecting portions be provided with a first differential pressure detecting portion and a second differential pressure detecting portion, and that the first differential pressure detecting portion have a measurement range that is on the low flow rate side of the second differential pressure detecting portion.

If this type of structure is employed, then by using the first differential pressure detecting section to measure the low flow rate side, and using the second differential pressure detecting section to measure the high flow rate side, it is possible to measure a broad range of flow rates highly accurately.

As an example of the specific structure of the first differential pressure detecting portion and the second differential pressure detecting portion, a structure in which the first differential pressure detecting portion and the second differential pressure detecting portion detect a differential pressure between a total pressure and a static pressure of the fluid body, and have a Pitot tube that is provided with total pressure holes that are used to detect the total pressure, and static pressure holes that are used to detect the static pressure may be considered. In this structure, in order to ensure that the measurement range of the first differential pressure detecting portion is on the low flow rate side, it is desirable that the total pressure holes and static pressure holes of the first differential pressure detecting portion be larger than the total pressure holes and static pressure holes of the second differential pressure detecting portion.

In order to enable the first differential pressure detecting portion to make highly accurate flow rate measurements in a measurement range on the low flow rate side, and to enable highly accurate flow rate measurements to be made by the second differential pressure detecting portion in a measurement range on the high flow rate side, it is desirable that there be provided a flow rate calculating unit that, when the flow rate of the fluid body which has been obtained using the first differential pressure detecting portion or the second differential pressure detecting portion is less than a predetermined value, outputs the flow rate of the fluid body obtained by the first differential pressure detecting portion as a measurement value, and when the flow rate of the fluid body which has been obtained using the first differential pressure detecting portion or the second differential pressure detecting portion is equal to or greater than a predetermined value, outputs the flow rate of the fluid body obtained by the second differential pressure detecting portion as a measurement value.

The measurement range on the low flow rate side is easily affected by pressure variations that are created by placing obstacles on the upstream side. Because of this, it is desirable that the first differential pressure detecting portion be provided on an upstream side of the flow path, and that the second differential pressure detecting portion be provided on a downstream side of the flow path.

If this type of structure is employed, then because this is not a structure in which a separate differential pressure detecting portion is disposed on the upstream side of the first differential pressure detecting portion, which has a measurement range on the low flow rate side, the measurements are not affected by pressure variations caused by a separate differential pressure detecting portion, so that it is possible for flow rates during low flow rate periods, such as when, for example, a vehicle is idling, to be accurately measured.

It is also desirable that the differential pressure flow meter be provided with an attachment pipe that is attached to an aperture portion of an exhaust pipe, and forms a flow path along which exhaust gas emitted from the exhaust pipe flows, and that the at least two differential pressure detecting portions be provided in this attachment pipe.

If this type of structure is employed, it is possible to measure a flow rate using two or more differential pressure detecting portions simply by attaching a single attachment pipe to an exhaust pipe.

It is also possible for the differential pressure flow meter of the present invention to be incorporated into an exhaust gas analysis device. In this case, it is desirable that the exhaust gas analysis device be provided with the above-described differential pressure flow meter, and with an exhaust gas analyzer that measures concentrations of predetermined components contained in the exhaust gas, and that an exhaust gas sampling unit that samples the exhaust gas and guides it to the exhaust gas analyzer be provided in the attachment pipe of the differential pressure flow meter.

Furthermore, a flow rate measurement method according to the present invention is a flow rate measurement method employing a differential pressure flow meter that detects differential pressures in a fluid body flowing along a flow path, and calculates a flow rate of the fluid body from those differential pressures, and that has a first differential pressure detecting portion and a second differential pressure detecting portion that each have mutually different measurement ranges, and in which the measurement range of the first differential pressure detecting portion is on the low flow rate side of the measurement range of the second differential pressure detecting portion, wherein, when the flow rate of the fluid body which has been obtained using the first differential pressure detecting portion or the second differential pressure detecting portion is less than a predetermined value, the flow rate of the fluid body obtained by the first differential pressure detecting portion is output as a measurement value, and when the flow rate of the fluid body which has been obtained using the first differential pressure detecting portion or the second differential pressure detecting portion is equal to or greater than a predetermined value, the flow rate of the fluid body obtained by the second differential pressure detecting portion is output as a measurement value.

[Effects of the Invention]

According to the present invention which has the above-described structure, because at least two differential pressure detecting portions having mutually different measurement ranges are provided, it is possible to measure a broad range of flow rates with a high degree of accuracy.

[Brief description of the drawings]

[FIG. 1] FIG. 1 is a schematic view showing the structure of an exhaust gas analysis device according to the present embodiment.
[FIG. 2] FIG. 2 is an enlarged cross-sectional view principally showing a tail pipe attachment portion of the same embodiment.
[FIG. 3] FIG. 3 is a layout diagram as seen from an upstream side in a flow path direction showing a positional relationship between differential pressure flow meters of the same embodiment.
[FIG. 4] FIG. 4 is a flowchart showing a flow rate measurement method of the same embodiment.
[FIG. 5] FIG. 5 is a layout diagram as seen from an upstream side in a flow path direction showing a positional relationship between differential pressure flow meters of a variant embodiment.
[FIG. 6] FIG. 6 is a cross-sectional view showing the structure of a differential pressure flow meter of a variant embodiment.

[Best Embodiments for Implementing the Invention]

Hereinafter, an embodiment of an exhaust gas analysis device that utilizes a differential pressure flow meter according to the present invention will be described with reference made to the drawings.

[Device structure]

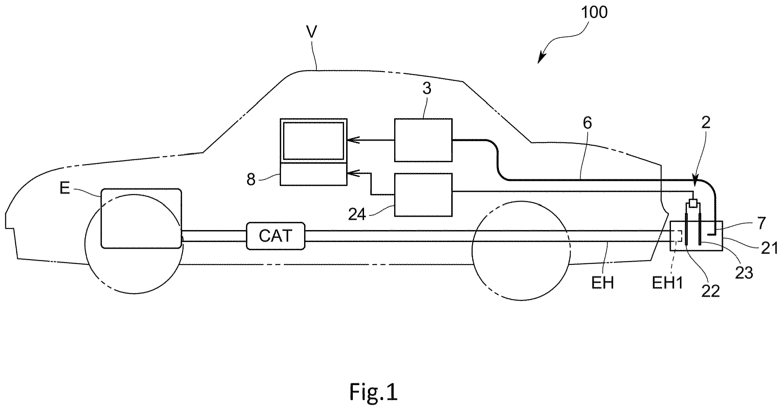

An exhaust gas analysis device 100 of the present embodiment is a vehicle-mounted type of exhaust gas analysis device that is mounted, for example, in a vehicle V, and analyzes in real time during on-road travel the exhaust gas that is emitted from an internal combustion engine E of the vehicle V while the vehicle V is traveling on a road. The exhaust gas analysis device 100 is a direct sampling type of analysis device that does not dilute captured exhaust gas, but instead measures the unmodified concentration thereof. Note that the exhaust gas analysis device 100 may also be a type of analysis device that analyzes in real time during simulated traveling the exhaust gas that is emitted from an internal combustion engine of a vehicle while this vehicle is performing simulated travel on a chassis dynamometer.
Specifically, as is shown in FIG. 1, this exhaust gas analysis device 100 is provided with a differential pressure flow meter 2 that is attached to an aperture portion EH1 of an exhaust pipe EH that is connected to the internal combustion engine E, and that measures a flow rate of exhaust gas emitted from this exhaust pipe EH, and with a gas analyzer 3 that is used to measure the concentrations of components needing to be measured that are contained in the exhaust gas emitted from the exhaust pipe EH.
The differential pressure flow meter 2 detects differential pressures in exhaust gas flowing along a flow path, and calculates the flow rate of the exhaust gas from these differential pressures. The differential pressure flow meter 2 is provided with an attachment pipe 21 that is attached to the exterior of the aperture portion EH1 of the exhaust pipe EH, and with a first differential pressure detecting portion 22 and a second differential pressure detecting portion 23 that are used to detect differential pressures in the exhaust gas flowing through the attachment pipe 21.
The attachment pipe 21 is formed as a straight pipe that is attached such that it covers an external circumferential surface of the aperture portion EH1 of the exhaust pipe EH. In the present embodiment, a round pipe having a circular cross-section is used for the attachment pipe 21. An aperture portion at one end of the attachment pipe 21 is fitted over the aperture portion EH1 of the exhaust pipe EH, while an aperture portion at another end thereof is left open. Exhaust gas is emitted to the outside via this other end aperture portion.
Moreover, in addition to the first differential pressure detecting portion 22 and the second differential pressure detecting portion 23, an exhaust gas temperature gauge 4 that detects a temperature $T_{exh}(t)$ of the exhaust gas, and an absolute pressure meter 5 that measures an exhaust gas pressure $P_{exh}(t)$ are also provided in the attachment pipe 21. Namely, the two differential pressure detecting portions 22 and 23 are served by the single exhaust gas temperature gauge 4 and the single absolute pressure meter 5. As a consequence, the structure of the attachment pipe 21 can be made simpler.

The first differential pressure detecting portion 22 and the second differential pressure detecting portion 23 detect a differential pressure ∆P between the total pressure and the static pressure of the exhaust gas. The first differential pressure detecting portion 22 and the second differential pressure detecting portion 23 have Pitot tubes 2P that are equipped with total pressure holes 2h1 that are used to detect the total pressure, and static pressure holes 2h2 that are used to detect the static pressure, and a differential pressure sensor 2S such as a differential pressure transmitter that detects the differential pressure ∆P between the total pressure and the static pressure of the exhaust gas via the Pitot tubes 2P.

The Pitot tubes 2P have a total pressure intake pipe portion 2P 1 in which the total pressure holes 2h1 are formed and that introduces the total pressure into the differential pressure sensor 2S, and a static pressure intake pipe portion 2P2 in which the static pressure holes 2h2 are formed and that introduces the static pressure into the differential pressure sensor 2S. In this example, the openings of the total pressure holes 2h1 face towards the upstream side of the flow path, while the openings of the static pressure holes 2h2 face towards the downstream side of the flow path on the opposite side from the total pressure holes 2h1. Note that in the present embodiment, a structure is employed in which the single differential pressure sensor 2S is shared by both the first differential pressure detecting portion 22 and the second differential pressure detecting portion 23, however, it is also possible to form a structure in which both the first differential pressure detecting portion 22 and the second differential pressure detecting portion 23 have their own individual differential pressure sensor 2S.

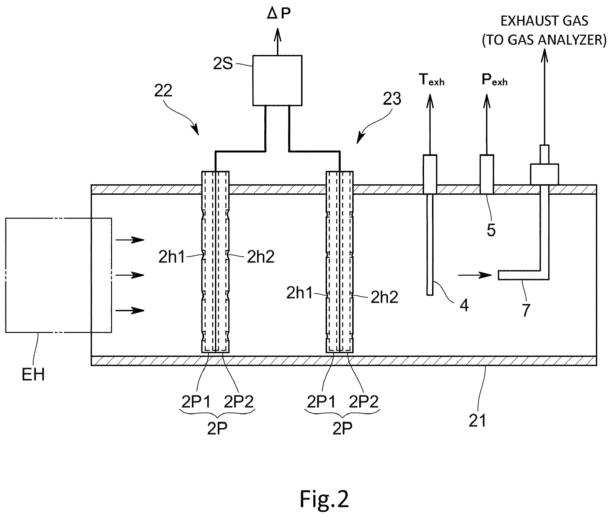

Here, the first differential pressure detecting portion 22 and the second differential pressure detecting portion 23 of the present embodiment have mutually different flow rate measurement ranges. Specifically, as is shown in FIG. 2, the first differential pressure detecting portion 22 has a measurement range on the low flow rate side of the second differential pressure detecting portion 23. More specifically, the total pressure holes 2h1 and the static pressure holes 2h2 of the first differential pressure detecting portion 22 are larger than the total pressure holes 2h1 and the static pressure holes 2h2 of the second differential pressure detecting portion 23. By making the total pressure holes 2h1 and the static pressure holes 2h2 of the first differential pressure detecting portion 22 larger in this way, a structure is created that makes it easier to receive pressure on the low pressure side in the first differential pressure detecting portion 22, and makes it possible to accurately detect low differential pressures. Note that the flow rate measurement range of the first differential pressure detecting portion 22 might be, for example, from 0~3 m$^3$/min, and the flow rate measurement range of the second differential pressure detecting portion 23 might be, for example, from 0~10 m$^3$/min. In the present embodiment, a case is shown in which one flow rate measurement range is contained within the other flow rate measurement range.

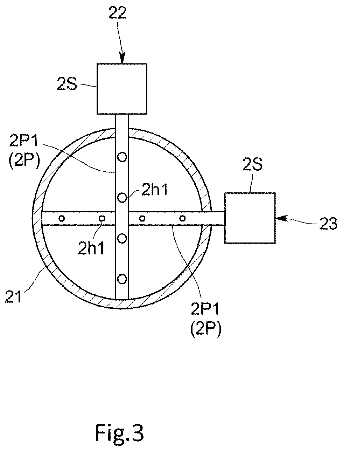

Moreover, in the present embodiment, the first differential pressure detecting portion 22 is provided on the upstream side of the flow path, while the second differential pressure detecting portion 23 is provided on the downstream side of the flow path (see FIG. 2). Specifically, the position where the Pitot tube 2P of the first differential pressure detecting portion 22 is inserted into the attachment pipe 21 is located on the upstream side of the position where the Pitot tube 2P of the second differential pressure detecting portion 23 is inserted into the attachment pipe 21. At this time, it is desirable to employ an arrangement in which the total pressure holes 2h1 and the static pressure holes 2h2 of the first differential pressure detecting portion 22 are not superimposed on top of the total pressure holes 2h1 and the static pressure holes 2h2 of the second differential pressure detecting portion 23 when viewed from the flow path direction. For example, as is shown in FIG. 3, by employing an arrangement in which the Pitot tube 2P of the first differential pressure detecting portion 22 extends orthogonally to the Pitot tube 2P of the second differential pressure detecting portion 23, it is possible to prevent the total pressure holes 2h1 and static pressure holes 2h2 of the respective Pitot tubes 2P from being mutually superimposed on each other.

Next, using the differential pressure ∆P obtained by the first differential pressure detecting portion 22 and the second differential pressure detecting portion 23, a flow rate calculating unit 24 of the differential pressure flow meter 2 calculates the flow rate of the exhaust gas.

Specifically, the flow rate calculating unit 24 uses the following formula to calculate a volumetric flow rate Q$_{exh}$(t) [m$^3$/min] of the exhaust gas in a standard state from the differential pressure ∆P from the differential sensor S2 obtained from at least one of the first differential pressure detecting portion 22 or the second differential pressure detecting portion 23, from the exhaust gas temperature T$_{exh}$(t) [K] obtained from the exhaust gas temperature gauge 4, and from the exhaust gas pressure P$_{exh}$(t) [kPa] obtained from the absolute pressure meter 5.

[Formula 1]

$$Q_{exh}(t) = k \times \sqrt{\frac{P_{exh}(t)}{P_0} \times \frac{T_0}{T_{exh}(t)} \times \frac{\Delta P}{\rho_{exh}}}$$

Wherein k = Proportionality coefficient

$P_0$ = Standard pressure (101.3 [kPa])

$T_0$ = Standard temperature (293.15 [K])

$\rho_{exh}$ = exhaust gas concentration [g/m$^3$] in a standard state.

Note that the proportionality coefficient K, the standard pressure $P_0$, the standard temperature To, and the exhaust gas concentration $\rho_{exh}$ are input in advance.

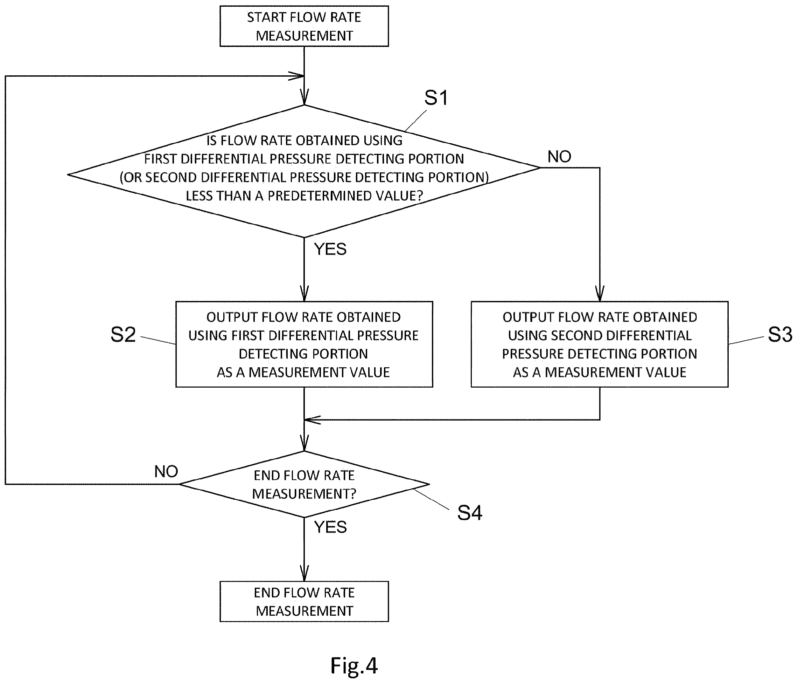

Here, as is shown in FIG. 4, the flow rate calculating unit 24 outputs the flow rate of the exhaust gas using, for example, the following procedure.

The flow rate calculating unit 24 determines whether or not the exhaust gas flow rate $Q_{exh}(t)$ obtained from the first differential pressure detecting portion 22 or the second differential pressure detecting portion 23 is less than a predetermined value (step S1). If the exhaust gas flow rate $Q_{exh}(t)$ is less than the predetermined value, then the flow rate calculating unit 24 outputs the exhaust gas flow rate $Q_{exh}(t)$ obtained from the first differential pressure detecting portion 22 as a measurement value (step S2). If, on the other hand, as a result of the determination made in step S1, the exhaust gas flow rate $Q_{exh}(t)$ is found to be greater than the predetermined value, the flow rate calculating unit 24 outputs the exhaust gas flow rate $Q_{exh}(t)$ obtained from the second differential pressure detecting portion 23 as the measurement value (step S3). The flow rate calculating unit 24 performs this processing sequence either continuously or at regular intervals until the flow rate measurement is ended (step S4).

Note that it is also possible to determine whether or not to switch the differential pressure detecting portion that is to be used by comparing the exhaust gas flow rate $Q_{exh}(t)$ with a flow rate measurement range. For example, the exhaust gas flow rate $Q_{exh}(t)$ may be compared with the flow rate measurement range of the first differential pressure detecting portion 22, and if the exhaust gas flow rate $Q_{exh}(t)$ is equal to or greater than an upper limit value of this flow rate measurement range or than a predetermined value in the vicinity of this upper limit value, then the differential pressure detecting portion to be used may be switched to the second differential pressure detecting portion 23. Alternatively, the exhaust gas flow rate $Q_{exh}(t)$ may be compared with the flow rate measurement range of the second differential pressure detecting portion 23, and if the exhaust gas flow rate $Q_{exh}(t)$ is equal to or less than a lower limit value of this flow rate measurement range or a second predetermined value in the vicinity of this lower limit value, then the differential pressure detecting portion to be used may be switched to the first differential pressure detecting portion 22. The aforementioned predetermined value in the vicinity of the upper limit value and predetermined value in the vicinity of the lower limit value can be set arbitrarily by a user.

Moreover, the gas analyzer 3 continuously measures the concentrations of components needing to be measured (for example, CO, $CO_2$, $NO_X$, and THC and the like) that are contained in the exhaust gas. Note that if the gas analyzer 3 is one that measures the concentrations of CO and $CO_2$, then an NDIR detector that employs a non-dispersive infrared absorption method (an NDIR method) can be used, while if the gas analyzer 3 is one that measures the concentration of $NO_X$, then a CLD detector that employs a chemiluminescence detection method (CLD) can be used. If the gas analyzer 3 is one that measures the concentration of THC, then an FID detector that employs a flame ionization detection method (FID) can be used. The gas analyzer 3 may be equipped with any one of these detectors, or may be equipped with any combination of these detectors. Additionally, the gas analyzer 3 may be one that uses a variety of analysis methods in accordance with the components to be measured.

An intake pipe 6 that is used to introduce sampled exhaust gas is connected to the gas analyzer 3. One end portion of this intake pipe 6 is connected to the gas analyzer 3, while an exhaust gas sampling unit 7 that samples exhaust gas is provided at another end portion of the intake pipe 6. The exhaust gas sampling unit 7 is provided in the above-described attachment pipe 21 of the differential pressure flow meter. This exhaust gas sampling unit 7 is formed by a sampling pipe that captures a portion of the exhaust gas flowing through the attachment pipe 21. Note that the exhaust gas sampling unit 7 is provided on the downstream side of the first and second differential pressure detecting portions 22 and 23 inside the attachment pipe 21, and does not affect the pressure detection performed by the first and second differential pressure detecting portions 22 and 23 such as by causing pressure variations or the like.

A concentration signal for each component acquired by the gas analyzer 3 is transmitted to a higher-order computing device 8, and is then used, together with flow rate signals output from the flow rate calculating unit 24 of the differential pressure flow meter 2, for the calculation of the emission mass of each component.

(Effects provided by the present embodiment)

According to the exhaust gas analysis device 100 according to the present embodiment that has the above-described structure, because the two differential pressure detecting portions 22 and 23 having mutually different flow rate measurement ranges are provided, it is possible to measure a broad range of flow rates with a high degree of accuracy. In particular, by using the first differential pressure detecting portion 22 to make the measurements on the low flow rate side and using the second differential pressure detecting portion 23 to make the measurements on the high flow rate side, it is possible to measure a broad range of flow rates with a high degree of accuracy.

Moreover, because this is not a structure in which a separate differential pressure detecting portion 23 is disposed on the upstream side of the first differential pressure detecting portion 22, which has a measurement range on the low flow rate side, the measurements made by the first differential pressure detecting portion 22 are not affected by pressure variations caused by a separate differential pressure detecting portion 23, so that it is possible for flow rates during low flow rate periods, such as when, for example, a vehicle is idling, to be accurately measured.

Furthermore, because the two differential pressure detecting portions 22 and 23 are provided in the single attachment pipe 21, it is possible to perform a flow rate measurement using the two differential pressure detecting portions 22 and 23 simply by attaching this attachment pipe 21 to the exhaust pipe EH. Here, because the exhaust gas sampling unit 7 is provided in the attachment pipe 21, attaching the exhaust gas sampling unit 7 can also be easily performed.

(Additional embodiments)

Note that the present invention is not limited to the above-described embodiment.

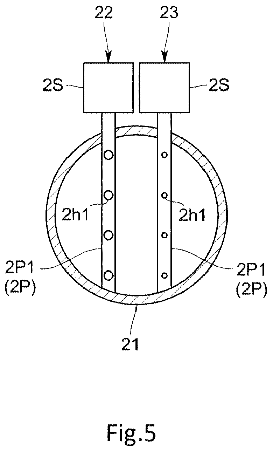

For example, in the above-described embodiment, the two differential pressure detecting portions 22 and 23 are disposed such that they mutually intersect each other when viewed from the flow path direction, however, as is shown in FIG. 5, it is also possible for the two to be disposed in such a way that they do not mutually intersect each other such as, for example, by disposing them in parallel with each other when viewed from the flow path direction. In this case, there are no particular restrictions regarding the positions of the first differential pressure detecting portion 22 and the second differential pressure detecting portion 23 in the flow path direction, and the two differential pressure detecting portions 22 and 23 may either be placed side-by-side, or the first differential pressure detecting portion 22 may be positioned on the downstream side of the second differential pressure detecting portion 23.

Moreover, in the above-described embodiment, a structure in which the two differential pressure detecting portions 22 and 23 are provided is described, however, it is also possible for three or more differential pressure detecting portions to be provided.

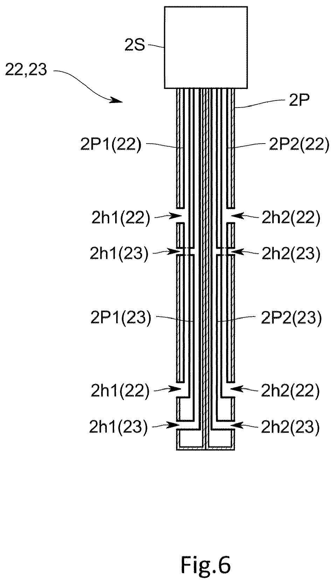

Furthermore, in the above described embodiment, a structure is employed in which the two differential pressure detecting portions 22 and 23 have mutually different Pitot tubes, however, as is shown in FIG. 6, it is also possible to provide the total pressure intake pipe portion 2P1 and the static pressure intake pipe portion 2P2 of the first differential pressure detecting portion 22, as well as the total pressure intake pipe portion 2P1 and the static pressure intake pipe portion 2P2 of the second differential pressure detecting portion 23 in the same single Pitot tube 2P. By doing this, the internal structure of the attachment pipe 21 can be simplified, pressure variations can be suppressed, and the measurement accuracy can be improved.

In the above-described embodiment a structure is employed in which at least two differential pressure detecting portions are provided in a single attachment pipe, however, if a plurality of attachment pipes are connected together in series then, in this structure, a single differential pressure detecting portion may be provided in each attachment pipe. Namely, the differential pressure flow meter may have two or more attachment pipes that are connected together in series, and a single differential pressure detecting portion is provided in each one of these attachment pipes.

In addition to this, in the above-described embodiment a case is described in which the differential pressure flow meter is used in an exhaust gas analysis device, however, this differential pressure flow meter may also be used in other types of analysis devices, or may be used by itself as an independent differential pressure flow meter.

Furthermore, it should be understood that the present invention is not limited to the above-described embodiments, and that various modifications and the like may be made thereto insofar as they do not depart from the spirit or scope of the present invention.

[Description of the Reference Numerals]

100 ...    Exhaust gas analysis device
2 ...       Differential pressure flow meter
21 ...    Attachment pipe
22 ...    First differential pressure detecting portion
23 ...    Second differential pressure detecting portion
2P ...    Pitot tube
2h1 ...   Total pressure holes
2h2 ...   Static pressure holes
24 ...    Flow rate calculating unit

3 ...      Exhaust gas analyzer
6 ...      Intake pipe
7 ...      Exhaust gas sampling unit
8 ...      Computing device

**Claims**

1. A differential pressure flow meter that detects differential pressures in a fluid body flowing along a flow path, and calculates a flow rate of the fluid body from those differential pressures comprising:

   at least two differential pressure detecting portions having mutually different measurement ranges.

2. The differential pressure flow meter according to claim 1, wherein the at least two differential pressure detecting portions are provided with a first differential pressure detecting portion and a second differential pressure detecting portion, and
   the first differential pressure detecting portion has a measurement range that is on the low flow rate side of the second differential pressure detecting portion.

3. The differential pressure flow meter according to claim 2, wherein the first differential pressure detecting portion and the second differential pressure detecting portion detect a differential pressure between a total pressure and a static pressure of the fluid body, and have a Pitot tube that is provided with total pressure holes that are used to detect the total pressure, and static pressure holes that are used to detect the static pressure, and
   the total pressure holes and static pressure holes of the first differential pressure detecting portion are larger than the total pressure holes and static pressure holes of the second differential pressure detecting portion.

4. The differential pressure detecting portion according to claim 2, wherein there is further provided a flow rate calculating unit that, when the flow rate of the fluid body which has been obtained using the first differential pressure detecting portion or the second differential pressure detecting portion is less than a predetermined value, outputs the flow rate of the fluid body obtained by the first differential pressure detecting portion as a measurement value, and when the flow rate of the fluid body which has been obtained using the first differential pressure detecting portion or the second differential pressure detecting portion is equal to or greater than a predetermined value, outputs the flow rate of the fluid body obtained by the second differential pressure detecting portion as a measurement value.

5. The differential pressure flow meter according to claim 2, wherein the first differential pressure detecting portion is provided on an upstream side of the flow path, and the second differential pressure detecting portion is provided on a downstream side of the flow path.

6. The differential pressure flow meter according to claim 1, wherein there is provided an attachment pipe that is attached to an aperture portion of an exhaust pipe, and forms a flow path along which exhaust gas emitted from the exhaust pipe flows, and
   the at least two differential pressure detecting portions are provided in the attachment pipe.

7. An exhaust gas analysis device comprising:

   the differential pressure flow meter according to claim 6; and
   an exhaust gas analyzer that measures concentrations of predetermined components contained in the exhaust gas, wherein
   an exhaust gas sampling unit that samples the exhaust gas and guides it to the exhaust gas analyzer is provided in the attachment pipe.

8. The exhaust gas analysis device according to claim 7, wherein this exhaust gas analysis device is capable of being mounted in a vehicle.

9. A flow rate measurement method employing a differential pressure flow meter that detects differential pressures in a fluid body flowing along a flow path, and calculates a flow rate of the fluid body from those differential pressures, and that has a first differential pressure detecting portion and a second differential pressure detecting portion that each have mutually different measurement ranges, and in which the measurement range of the first differential pressure detecting portion is on the low flow rate side of the measurement range of the second differential pressure detecting portion, wherein,

when the flow rate of the fluid body which has been obtained using the first differential pressure detecting portion or the second differential pressure detecting portion is less than a predetermined value, the flow rate of the fluid body obtained by the first differential pressure detecting portion is output as a measurement value, and when the flow rate of the fluid body which has been obtained using the first differential pressure detecting portion or the second differential pressure detecting portion is equal to or greater than a predetermined value, the flow rate of the fluid body obtained by the second differential pressure detecting portion is output as a measurement value.

Fig.1

EP 3 333 550 A1

EXHAUST GAS
(TO GAS ANALYZER)

$P_{exh}$

$T_{exh}$

7

5

4

21

23

2h2

2h1

2P1 2P2
2P

$\Delta P$

2S

22

2h2

2h1

2P1 2P2
2P

EH

Fig.2

Fig.3

START FLOW RATE
MEASUREMENT

S1

IS FLOW RATE OBTAINED USING
FIRST DIFFERENTIAL PRESSURE DETECTING PORTION
(OR SECOND DIFFERENTIAL PRESSURE DETECTING PORTION)
LESS THAN A PREDETERMINED VALUE?

NO

YES

S2

OUTPUT FLOW RATE OBTAINED
USING FIRST DIFFERENTIAL PRESSURE
DETECTING PORTION
AS A MEASUREMENT VALUE

OUTPUT FLOW RATE OBTAINED
USING SECOND DIFFERENTIAL
PRESSURE DETECTING PORTION
AS A MEASUREMENT VALUE

S3

NO

END FLOW RATE
MEASUREMENT?

S4

YES

END FLOW RATE
MEASUREMENT

Fig.4

EP 3 333 550 A1

13

Fig.5

Fig.6

Europäisches
Patentamt

European
Patent Office

Office européen
des brevets

# EUROPEAN SEARCH REPORT

Application Number

EP 17 18 4563

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/49284 A1 (YORK INT CORP [US]) 30 September 1999 (1999-09-30) * page 3, line 7 - line 15 * * page 4, line 29 - line 31 * * page 5, line 8 - line 10 * * figures 1, 5, 6 * * page 4, line 12 - line 14 * * page 6, line 1 - line 15 * * page 5, line 2 - line 3 * ----- | 1-9 | INV. G01F1/34 G01F7/00 G01F1/46 |
| A | R Pemberton: "AN OVERVIEW OF DYNAMIC PRESSURE MEASUREMENT CONSIDERATIONS", , 15 March 2010 (2010-03-15), pages 1-13, XP055452509, 1722 North Madson Street, Liberty Lake, WA 99019, United States Retrieved from the Internet: URL:http://scanivalve.com/media/6930/pneum atic_frequency_response.pdf [retrieved on 2018-02-19] * page 5, line 16 - line 21 * ----- | 3 | |
| A | EP 1 405 989 A2 (HORIBA LTD [JP]) 7 April 2004 (2004-04-07) * figures 2, 17, 18 * * paragraph [0044] * ----- | 6-8 | TECHNICAL FIELDS SEARCHED (IPC) G01F |
| A | EP 1 793 210 A2 (HORIBA LTD [JP]) 6 June 2007 (2007-06-06) * figure 11 * ----- | 1-9 | |
| A | GB 1 080 143 A (WILLIAM YUNG LING MA PH D B SC) 23 August 1967 (1967-08-23) * figure 2 * ----- | 1-9 | |

The present search report has been drawn up for all claims

| Place of search | Date of completion of the search | Examiner |
|---|---|---|
| The Hague | 20 February 2018 | Régert, Tamás |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 18 4563

20-02-2018

| Patent document cited in search report | | Publication date | Patent family member(s) | | Publication date |
|---|---|---|---|---|---|
| WO 9949284 | A1 | 30-09-1999 | AU | 3097199 A | 18-10-1999 |
| | | | TW | 376437 B | 11-12-1999 |
| | | | US | 6079627 A | 27-06-2000 |
| | | | WO | 9949284 A1 | 30-09-1999 |
| EP 1405989 | A2 | 07-04-2004 | DE | 60312940 T2 | 17-01-2008 |
| | | | EP | 1405989 A2 | 07-04-2004 |
| | | | US | 2004064243 A1 | 01-04-2004 |
| EP 1793210 | A2 | 06-06-2007 | DE | 602004004709 T2 | 22-11-2007 |
| | | | EP | 1508788 A1 | 23-02-2005 |
| | | | EP | 1793210 A2 | 06-06-2007 |
| | | | EP | 3190393 A1 | 12-07-2017 |
| | | | US | 2005080550 A1 | 14-04-2005 |
| GB 1080143 | A | 23-08-1967 | NONE | | |

EPO FORM P0459

For more details about this annex : see Official Journal of the European Patent Office, No. 12/82

**EP 3 333 550 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004144574 A